(19) Europäisches Patentamt
European Patent Office
Office européen des brevets

(11) Publication number: 0 065 800 B1

(12) EUROPEAN PATENT SPECIFICATION

(45) Date of publication of patent specification: 28.08.85

(51) Int. Cl.$^4$: C 12 N 9/96

(21) Application number: 82200579.9

(22) Date of filing: 12.05.82

# (54) Enzyme solutions and method for the preparation thereof.

(30) Priority: 13.05.81 NL 8102346

(43) Date of publication of application:
01.12.82 Bulletin 82/48

(45) Publication of the grant of the patent:
28.08.85 Bulletin 85/35

(84) Designated Contracting States:
AT BE CH DE FR GB IT LI LU NL SE

(56) References cited:
DE-A-2 807 090
GB-A-1 120 298
US-A-1 444 250
US-A-2 475 792

CHEMICAL ABSTRACTS, vol. 75, no. 7, 16th August 1971, page 246, no. 47618w, Columbus, Ohio, USA, J.W. DONOVAN et al.: "Beta-N-Acetylglucoseaminidase activity of egg white. I. Kinetics of the reaction and determination of the factors affecting the stability of the enzyme in egg white"

(73) Proprietor: NEDERLANDSE CENTRALE ORGANISATIE VOOR TOEGEPAST-NATUURWETENSCHAPPELIJK ONDERZOEK
Juliana van Stolberglaan 148
NL-2595 CL The Hague (NL)

(72) Inventor: Annokkée, Gustaaf Johannus
Keizersmantel 16
NL-7361 ZN Beekbergen (NL)
Inventor: ter Meulen, Berend Philippus
Cornelis Hendrixstraat 90
NL-7371 AV Loenen (Gld.) (NL)

(74) Representative: Plaisier, Aart et al
Nederlandsch Octrooibureau Johan de Wittlaan 15 P.O. Box 29720
NL-2502 LS 's-Gravenhage (NL)

(56) References cited:
DERWENT JAPANESE PATENTS REPORT, vol. R, no. 37, 6th November 1970, abstract no. 65413R, London, G.B.

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 065 800 B1

Courier Press, Leamington Spa, England.

**Description**

The invention relates to stabilized enzyme solutions, to a method for the preparation thereof, and to a method for carrying out an enzymatic process.

It is known that the stability of enzymes in solution may be improved by addition of protective colloids generally, and especially by addition of albumin, a protein from egg white, blood serum or milk.

DE—A—2 807 090 describes lipase preparations containing globular proteins of animal origin as stabilizers. The use of albumin is preferred, but mixtures of proteins may be used as well. Specific mixtures are not mentioned, however.

According to the present invention it has been found that the complete, undenatured white fraction of eggs (egg white) is an excellent stabilizer for enzymes, such as oxidizing, reducing and hydrolyzing enzymes, and is a better stabilizer than the more or less purified globular proteins suggested in DE—A—2 807 090. More especially, the stabilizing activity of undenatured egg white is surprisingly better than that of egg albumin. The present finding cannot be used for the stabilization of proteolytic enzymes, since these would attack the proteins present in egg white. Also, the invention does not relate to the stabilization of enzymes naturally occurring in undenatured egg white.

In the first place, the invention provides an enzyme solution comprising one or more non-proteolytic enzymes not normally present in undenatured egg white and, as a stabilizer, undenatured egg white. The white fraction of chicken eggs has been found to be particularly suitable, although any other egg white, such as egg white from duck or goose eggs, may be used as well.

Stable enzyme solutions are of importance for various applications, such as for example the conversion of macromolecular substrates, especially in enzyme reactors, such as membrane reactors in which the membrane is only permeable for the product formed.

The invention also relates to a method for stabilizing enzymes, wherein one or more non-proteolytic enzymes not normally present in undenatured egg white are dissolved in a solvent consisting of or comprising undenatured egg white, preferably chicken egg white.

Moreover the invention relates to a method for carrying out an enzymatic process, wherein a stabilizing enzyme solution according to the invention or a stabilized enzyme solution obtained by the method of the invention is used as a catalyst.

The invention is further elucidated by the following examples, wherein undenatured white fractions of chicken eggs, goose eggs and duck eggs, respectively, have been used.

Example I
Hydrolysis of lactose by means of lactase

$$\text{Lactose} \xrightarrow{\text{lactase}} \text{glucose+galactose.}$$

Lactase (β-galactosidase) in a quantity of 250 mg was dissolved in 50 ml of each of the white fractions of chicken eggs, duck eggs and goose eggs respectively, all neutralized to pH 7 with lactic acid. Some drops of toluene were added for protection against microbial infection, and the solutions were stored at 5°C. With intervals of one week each time 0.5 ml of these enzyme solutions were mixed with 25 ml of lactose solution (5 g lactose, dissolved in a mixture of 90 ml demineralized water and 10 ml of a 0.05 molar phosphate buffer, pH 7). After reacting for 5 hours at 25°C the conversion in all cases was determined by means of an Autoanalyzer method, whereby the total reducing capacity was measured.

For comparison, a solution of 250 mg lactase in 50 ml of a 0.05 molar phosphate buffer, pH 7, was prepared and stored under the same conditions and sampled and measured weekly.

Table A shows the activity of the lactase solutions after various storage periods, expressed as percentage of lactose converted in 5 hours.

TABLE A

| Storage period in days | Conversion measured after 5 hours: Lactase in white of egg: chicken | goose | duck | Lactase in phosphate buffer |
|---|---|---|---|---|
| 0 | 55 | 50 | 52 | 60 |
| 14 | 55 | 50 | 52 | 40 |
| 21 | 55 | 50 | 52 | 32 |
| 28 | 55 | 50 | 52 | 25 |
| 35 | 55 | 49 | 51 | 15 |
| 42 | 55 | 49 | 51 | 15 |
| 49 | 55 | 49 | 50 | 12 |
| 56 | 55 | 48 | 50 | 10 |

It appears from the above Table that the activity of lactase in the white fractions of chicken, goose and duck eggs, respectively, is maintained, whereas that of lactase in phosphate buffer decreases to only one sixth of the original activity after eight weeks.

Example II

Oxidation of ethanol to ethanal by means of alcohol dehydrogenase (ADH)

A quantity of 2 mg of ADH was dissolved in 10 ml of the white fraction of chicken eggs, neutralized to pH 7. This solution was stored at 5°C and, with regular intervals, a quantity of 0.2 ml thereof was added to a mixture of 0.2 ml of $NAD^+$ solution (0.01 mmol $NAD^+$/10 ml), 0.1 ml ethanol and 2 ml of 0.1 molar phosphate buffer solution with pH 7.0. This solution in its entirety was quickly homogenized and subsequently the enzyme activity was determined by measuring the conversion of $NAD^+$ into NADH at 340 nm.

For comparison identical quantities of ADH were also dissolved in 10 ml of a 0.1 molar phosphate buffer, pH 7, with addition of various quantities of albumin. These solutions were sampled and measured for stability of the enzyme in the manner described above.

In order to prevent microbial infections in the enzyme solutions to be tested, a small quantity of butyl acetate was added thereto before storage.

The measurements of the activity were carried out at 20°C. The results obtained are summarized in Table B.

TABLE B
Measured initial activity of ADH (in % of the initial activity at day 0)

| Storage time in days | ADH in white of chicken eggs | ADH in phosphate buffer +x mg/10 ml albumin x=0 | 0.5 | 1.0 | 2.0 | 5.0 |
|---|---|---|---|---|---|---|
| 0 | 100 | 100 | 100 | 100 | 100 | 100 |
| 1 | 98 | 96 | 96 | 96 | 96 | 96 |
| 2 | 96 | 94 | 90 | 92 | 92 | 91 |
| 5 | 91 | 90 | 85 | 86 | 86 | 87 |
| 10 | 80 | 75 | 78 | 80 | 81 | 80 |
| 15 | 72 | 67 | 52 | 55 | 58 | 59 |
| 20 | 60 | 56 | 34 | 40 | 40 | 43 |
| 30 | 45 | 30 | 8 | 12 | 13 | 14 |

From this Table it appears clearly that the stability of ADH is substantially improved by the white fraction of chicken eggs.

Example III
Reduction of cortisone to 20 β-dihydrocortisone by means of 20 β-hydroxysteroid dehydrogenase (20 β-HSDH)

A quantity of 0.9 mg of 20 β-HSDH was dissolved in 10 ml of the white fraction of chicken eggs, neutralized to pH 7. This solution was stored at 5°C and, at regular intervals, a quantity of 0.1 ml of this solution was added to a mixture of 0.2 ml of NADH solution, comprising 0.01 mmole of NADH/10 ml, and 2 ml of a 0.1 molar phosphate buffer solution with pH 7 comprising cortisone dissolved therein in a concentration of 150 mg/l. This solution was quickly homogenized in its entirety and, subsequently, the enzyme activity was measured by determining the conversion of NADH into $NAD^+$ at 340 nm.

For comparison, an identical quantity of 20 β-HDSH was dissolved in 10 ml of a 0.1 molar phosphate buffer with pH 7 to which various quantities of albumin were added. The activity was determined in the manner described above.

In order to prevent microbial infections in the solutions to be tested, a small quantity of butyl acetate was added prior to storage.

All measurements of activity were carried out at 20°C. The results obtained are summarized in Table C below.

TABLE C
Measured initial activity of 20 β-HSDH
(in % of the initial activity at day 0)

| Storage time in days | 20 β-HSDH in white of chicken eggs | 20 β-HSDH in phosphate buffer +x mg/10 ml albumin x=0 | 0.5 | 1.0 | 2.0 | 5.0 |
|---|---|---|---|---|---|---|
| 0 | 100 | 100 | 100 | 100 | 100 | 100 |
| 1 | 98 | 53 | 94 | 94 | 96 | 95 |
| 2 | 96 | 38 | 90 | 91 | 93 | 93 |
| 5 | 92 | 9 | 65 | 66 | 68 | 68 |
| 10 | 88 | 1 | 32 | 34 | 36 | 38 |
| 15 | 83 | 0 | 12 | 18 | 19 | 22 |
| 22 | 79 | 0 | 3 | 2 | 8 | 14 |
| 30 | 70 | 0 | 1 | 0 | 1 | 4 |

From this Table it appears that the stability of 20 β-HSDH is clearly improved by the white fraction of chicken eggs.

Example IV

Reduction of cortisone to 20 β-dihydrocortisone by means of 20 β-HSDH with regeneration of the co-enzyme NADH from $NAD^+$.

An enzyme solution was prepared by dissolving a suspension of 50 μm 20 β-HSDH (0.5 units), 5 mg ADH (2000 units) and 5 mg $NAD^+$ in 10 ml of the white fraction of chicken eggs, neutralized to pH 7 with lactic acid, at 25°C, and was stored at said temperature. In order to prevent microbial infection, a few drops of toluene were added. In order to determine the activity as a function of time (the temperature being kept at 25°C during the determinations) 0.5 ml of this enzyme solution was mixed each time with 25.0 ml of substrate solution. The composition of the substrate solution was: 0.1 g of cortisone+10 ml of ethanol per liter of demineralized water.

For comparison a similar enzyme/co-enzyme solution was prepared in 10 ml of a 0.05 molar phosphate buffer with pH 7.0 at 25°C, and was stored at the same temperature. In a similar way the activity as a function of time at a temperature of 25°C was determined for this solution.

The results are summarized in Table D.

TABLE D

| Storage time in days | Conversion after 5 hours (in %, determination by TLC-analysis) In white fraction of eggs | In phosphate buffer |
|---|---|---|
| 0 | 100 | 100 |
| 5 | 97 | 25 |
| 12 | 80 | 10 |
| 19 | 50 | 5 |
| 26 | 40 | 0 |
| 33 | 30 | 0 |

The Table clearly shows the stabilizing effect of the white fraction of chicken eggs for the relevant enzyme system.

## Claims

1. An enzyme solution comprising one or more non-proteolytic enzymes not normally present in undenatured egg white and, as a stabilizer, undenatured egg white.

2. The enzyme solution according to claim 1, in which the undenatured egg white is chicken egg white.

3. A method for stabilizing enzymes wherein one or more non-proteolytic enzymes not normally present in undenatured egg white are dissolved in a solvent consisting of or comprising undenatured egg white.

4. The method of claim 3 in which the egg white is chicken egg white.

5. A method for carrying out an enzymatic process, characterized by using an enzyme solution according to claim 1 or 2 or an enzyme solution obtained by the process of claim 3 or 4, as a catalyst.

## Patentansprüche

1. Enzym-Lösung bestehend aus einem oder mehreren nicht-proteolytischen Enzymen, die normalerweise in undenaturiertem Eiweiß nicht vorhanden sind, und undenaturiertes Eiweiß als Stabilisator.

2. Enzym-Lösung nach Anspruch 1, bei der das undenaturierte Eiweiß Hühnereiweiß ist.

3. Verfahren zur Stabilisierung von Enzymen, bei dem ein oder mehrere nicht-proteolytische Enzyme, die normalerweise in undenaturiertem Eiweiß nicht vorhanden sind, in einem Lösungsmittel gelöst werden, das aus undenaturiertem Eiweiß besteht oder dieses enthält.

4. Verfahren nach Anspruch 3, bei dem das Eiweiß Hünereiweiß ist.

5. Verfahren zur Ausführung eines enzymatischen Prozesses, gekennzeichnet durch die Verwendung einer Enzymlösung nach Anspruch 1 oder 2 oder einer Enzymlösung erhalten nach dem Verfahren gemäß einem der Ansprüche 3 oder 4 als Katalysator.

## Revendications

1. Une solution d'enzyme comprenant une ou plusieurs enzymes non protéolytiques qui ne sont normalement pas présentes dans le blanc d'oeuf non dénaturé et, comme stabilisant, du blanc d'oeuf non dénaturé.

2. La solution d'enzyme selon la revendication 1, dans laquelle le blanc d'oeuf non dénaturé est du blanc d'oeuf de poule.

3. Un procédé pour stabiliser les enzymes dans lequel une ou plusieurs enzymes non protéolytiques qui ne sont pas normalement présentes dans le blanc d'oeuf non dénaturé, sont dissoutes dans un solvent constitué en partie ou en totalité de blanc d'oeufs non dénaturés.

4. Le procédé de la revendication 3, dans lequel le blanc d'oeuf est du blanc d'oeuf de poule.

5. Un procédé pour réaliser un processus enzymatique caractérisé par l'emploi d'une solution d'enzyme selon la revendication 1 ou 2, ou d'une solution d'enzyme obtenue selon le procédé de la revendication 3 ou 4 comme catalyseur.